(19) **Europäisches Patentamt / European Patent Office / Office européen des brevets**

(11) **EP 4 549 559 A1**

(12) # EUROPEAN PATENT APPLICATION
published in accordance with Art. 153(4) EPC

(43) Date of publication:
07.05.2025 Bulletin 2025/19

(21) Application number: 23829377.3

(22) Date of filing: 30.01.2023

(51) International Patent Classification (IPC):
$C12N\ 9/16^{(2006.01)}$   $C12N\ 15/55^{(2006.01)}$
$C12N\ 15/81^{(2006.01)}$   $C12N\ 1/19^{(2006.01)}$
$C12R\ 1/84^{(2006.01)}$

(52) Cooperative Patent Classification (CPC):
Y02E 50/10

(86) International application number:
PCT/CN2023/073753

(87) International publication number:
WO 2024/001184 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.06.2022 CN 202210739640

(71) Applicant: INSTITUTE OF ANIMAL SCIENCE OF
CHINESE ACADEMY OF
AGRICULTURAL SCIENCES
Beijing 100193 (CN)

(72) Inventors:
• HUANG, Huoqing
  Beijing 100193 (CN)
• TU, Tao
  Beijing 100193 (CN)
• WANG, Qian
  Beijing 100193 (CN)
• YAO, Bin
  Beijing 100193 (CN)
• LUO, Huiying
  Beijing 100193 (CN)
• BAI, Yingguo
  Beijing 100193 (CN)

• WANG, Yuan
  Beijing 100193 (CN)
• SU, Xiaoyun
  Beijing 100193 (CN)
• WANG, Yaru
  Beijing 100193 (CN)
• ZHANG, Jie
  Beijing 100193 (CN)
• QIN, Xing
  Beijing 100193 (CN)
• WANG, Xiaolu
  Beijing 100193 (CN)
• ZHANG, Honglian
  Beijing 100193 (CN)
• YU, Huimin
  Beijing 100193 (CN)

(74) Representative: Forresters IP LLP
Skygarden
Erika-Mann-Straße 11
80636 München (DE)

Remarks:
The complete document including Reference
Table(s) and the Sequence Listing(s) can be
downloaded from the EPO website

(54) ## METHOD FOR IMPROVING THERMOSTABILITY OF PHYTASE, MUTANT AND USE

(57) The present invention relates to the field of genetic engineering, particularly to method for improving thermo-stability of phytase, mutant and use.The present invention introduces a series of mutations to the phytase APPAmut4, which may involve introducing disulfide bonds, reducing the free energy of unfolding, optimizing the key residues in the coevolution process, and significantly improving the thermal stability of the phytase.

Among the mutants of the present invention, the optimal mutant APPAmut9 retains about 70% of its activity after being treated for 5 minutes at 100 °C, while the phytase APPAmut4 has already been inactivated. Therefore, the present invention overcomes the shortcomings of the prior art and provides phytase mutants with high thermal stability suitable for wide application in fields such as energy, food, and feed.

Fig.1

**Description**

**FIELD OF THE INVENTION**

The present invention relates to the field of genetic engineering, particularly to method for improving thermo-stability of phytase, mutant and use.

**BACKGROUND OF THE INVENTION**

Phytase is an important industrial enzyme that can hydrolyze phytic acid into phosphoric acid residues. Phytase has been widely applied in various industries such as food processing, environmental protection, feed additive, and biofuel production. The heat resistance of phytase has become one of the bottleneck issues limiting its industrial application in the light of the requirement of the instantaneous high temperature during the process of feed granulation.

The formation of the disulfide bonds in proteins is an oxidation process that creates a covalent bond connecting the sulfur atoms of two cysteine residues, fixing the protein structure and stabilizing its active conformation, thereby contributing to its activity and stability. Therefore, the disulfide bond engineering is an effective strategy to improve the thermal stability of the protein. However, not all introduction of the disulfide bonds can improve the thermal stability. For example, Liu et al. found that when seven pairs of cysteine were introduced into alkaline $\alpha$-amylase derived from *Alkalinimonas amylolytica*, only three pairs, P35C-G426C, G116C-Q120C, and R436C-M480C, resulted in enhanced thermal stability of the enzyme, with the half lives at 60 °C increased by 4.1, 7.4, and 2.2 minutes, respectively, as compared to that of the wild type, while the introduction of the remaining four pairs of cysteine actually reduced thermal stability (LONG et al., 2014). Therefore, how to select appropriate mutation sites to introduce disulfide bonds is a key issue in improving enzyme thermal stability. The unfolding free energy $\Delta$ Gu reflects the difference in Gibbs free energy between the folding and unfolding states of a protein, which is an important indicator of protein thermal stability. It can be observed that some amino acid residues are highly correlated with each other rather than independent in multi-sequence alignment, which is manifested as a compensatory phenomenon in the process of evolution that the related residue compensate for the adverse effect when a certain amino acid of a protein undergoes a mutation, thereby maintaining the structure and function of the protein, and also named as a coevolution phenomenon that it's very important for maintaining the stability of the protein that the coevolutionary residues with correlation are adjacent in spatial structure(SUTTO et al., 2015).

**Order of the invention**

One order of the present invention is to provide phytase mutant having the improved thermal stability of the phytase APPAmut4 derived from *Yersinia intermedia*.

Another order of the present invention is to provide a gene encoding the above phytase mutant.

Another order of the present invention is to provide a recombinant vector comprising the above gene encoding the above phytase mutant.

Another order of the present invention is to provide a recombinant strain comprising the above gene encoding the above phytase mutant.

Another order of the present invention is to provide a method of preparing a phytase having the improved thermal stability.

Another order of the present invention is to provide a use of the above phytase mutant.

**SUMMARY OF THE INVENTION**

One aspect of the present invention is to provide a phytase mutant having the improved thermal stability by performing mutation to the phytase APPAmut4 comprising an amino acid sequence of SEQ ID NO:1, derived from *Yersinia intermedia.*

The present invention provides a phytase mutant having phytase activity and comprising the amino acid sequence of SEQ ID NO:1, except A corresponding to position 57 and A corresponding to position 103 of SEQ ID NO: 1 are replaced with C, and C respectively (i.e, mutant A57C/A103C), or,

> G corresponding to position 101 and V corresponding to position 116 of SEQ ID NO:1 are replaced with C, and C respectively (i.e. mutant G101C/V116C), or
>
> R corresponding to position 271 and E corresponding to position 413 of SEQ ID NO:1 are replaced with C, and C respectively (i.e. mutant R271C/E413C), or
>
> R corresponding to position 353 and L corresponding to position 401 of SEQ ID NO:1 are replaced with C, and C respectively (i.e. mutant R353C/L401C), or

A corresponding to position 147 and Y corresponding to position 268 of SEQ ID NO:1 are replaced with C, and C respectively (i.e. mutant A147C/Y268C), and wherein the phytase mutant has improved thermal stability as compared to a phytase consisting of the amino acid sequence of SEQ ID NO:1.

The phytase mutant according to the present invention is the phytase mutant APPAmut5 having phytase activity and comprising the amino acid sequence of SEQ ID NO: 1, except A corresponding to position 57, A corresponding to position 103, G corresponding to position 101, V corresponding to position 116, R corresponding to position 271, E corresponding to position 413, R corresponding to position 353, L corresponding to position 401, A corresponding to position 147, Y corresponding to position 268 of SEQ ID NO: 1 are replaced with C respectively, and wherein the phytase mutant has improved thermal stability as compared to a phytase consisting of the amino acid sequence of SEQ ID NO: 1.

The phytase mutant APPAmut5 has an amino acid sequence of SEQ ID NO: 2.

Further, the present invention provides the phytase mutant having phytase activity and comprising the amino acid sequence of SEQ ID NO:1, except G corresponding to position 65 is replaced with R, or,

E corresponding to position 282 is replaced with L, or,
G corresponding to position 365 is replaced with D, or,
D corresponding to position 133 is replaced with E, or,
R corresponding to position 382 is replaced with I, or,
S corresponding to position 393 is replaced with I, and wherein the phytase mutant has improved thermal stability as compared to a phytase consisting of the amino acid sequence of SEQ ID NO: 1.

The phytase mutant according to the present invention is the phytase mutant APPAmut8 having phytase activity and comprising the amino acid sequence of SEQ ID NO: 2, except G corresponding to position 65 is replaced with R, E corresponding to position 282 is replaced with L, and G corresponding to position 365 is replaced with D, and wherein the phytase mutant has improved thermal stability as compared to a phytase consisting of the amino acid sequence of SEQ ID NO: 2.

The phytase mutant APPAmut8 has an amino acid sequence of SEQ ID NO: 3.

The phytase mutant according to the present invention is the phytase mutant APPAmut9 having phytase activity and comprising the amino acid sequence of SEQ ID NO:3, except D corresponding to position 133 is replaced with E, R corresponding to position 382 is replaced with I, and S corresponding to position 393 is replaced with I, and wherein the phytase mutant has improved thermal stability as compared to a phytase consisting of the amino acid sequence of SEQ ID NO: 3.

The phytase mutant APPAmut9 has an amino acid sequence of SEQ ID NO: 4.

Another aspect of the present invention is provide genes encoding the above mutants.

The gene encoding the said phytase mutant APPAmut5 of the present invention comprises the nucleotide sequence of SEQ ID NO: 5.

The gene encoding the said phytase mutant APPAmut8 of the present invention comprises the nucleotide sequence of SEQ ID NO: 6.

The gene encoding the said phytase mutant APPAmut9 of the present invention comprises the nucleotide sequence of SEQ ID NO: 7.

Another aspect of the present invention is to provide a method of improving the thermal stability of a phytase comprising an amino acid of SEQ ID NO:1, wherein said method comprises the step of

replacing A corresponding to position 57 of SEQ ID NO:1 with C, and A corresponding to position 103 of SEQ ID NO:1 with C, or,
replacing G corresponding to position 101 of SEQ ID NO:1 with C, and V corresponding to position 116 of SEQ ID NO:1 with C, or,
replacing R corresponding to position 271 of SEQ ID NO:1 with C, and E corresponding to position 413 of SEQ ID NO:1 with C, or,
replacing R corresponding to position 353 of SEQ ID NO:1 with C, and L corresponding to position 401 of SEQ ID NO:1 with C, or,
replacing A corresponding to position 147 of SEQ ID NO:1 with C, and Y corresponding to position 268 of SEQ ID NO:1 with C; or
comprising the step of replacing G corresponding to position 65 with R, or,
replacing E corresponding to position 282 of SEQ ID NO: 1 with L, or,
replacing G corresponding to position 365 of SEQ ID NO: 1 with D, or,
replacing D corresponding to position 133 of SEQ ID NO: 1 with E, or,
replacing R corresponding to position 382 of SEQ ID NO: 1 with I, or,

replacing S corresponding to position 393 of SEQ ID NO: 1 with I.

Another aspect of the present invention is to provide a method of improving the thermal stability of a phytase comprising an amino acid of SEQ ID NO:1, wherein said method comprises the steps of replacing A corresponding to position 57 of SEQ ID NO:1with C, and A corresponding to position 103 of SEQ ID NO:1 with C,

replacing G corresponding to position 101 of SEQ ID NO:1 with C, and V corresponding to position 116 of SEQ ID NO:1 with C,
replacing R corresponding to position 271 of SEQ ID NO:1 with C, and E corresponding to position 413 of SEQ ID NO:1 with C,
replacing R corresponding to position 353 of SEQ ID NO:1 with C, and L corresponding to position 401 of SEQ ID NO:1 with C, and
replacing A corresponding to position 147 of SEQ ID NO:1 with C, and Y corresponding to position 268 of SEQ ID NO:1 with C.

Another aspect of the present invention is to provide a method of improving the thermal stability of a phytase comprising an amino acid of SEQ ID NO:1, wherein said method comprises the steps of replacing A corresponding to position 57 of SEQ ID NO:1with C, and A corresponding to position 103 of SEQ ID NO:1 with C,

replacing G corresponding to position 101 of SEQ ID NO:1 with C, and V corresponding to position 116 of SEQ ID NO:1 with C,
replacing R corresponding to position 271 of SEQ ID NO:1 with C, and E corresponding to position 413 of SEQ ID NO:1 with C,
replacing R corresponding to position 353 of SEQ ID NO:1 with C, and L corresponding to position 401 of SEQ ID NO:1 with C,
replacing A corresponding to position 147 of SEQ ID NO:1 with C, and Y corresponding to position 268 of SEQ ID NO:1 with C,
replacing G corresponding to position 65 with R,
replacing E corresponding to position 282 of SEQ ID NO: 1 with L, and
replacing G corresponding to position 365 of SEQ ID NO: 1 with D.

Another aspect of the present invention is to provide a method of improving the thermal stability of a phytase comprising an amino acid of SEQ ID NO:1, wherein said method comprises the step of replacing A corresponding to position 57 of SEQ ID NO:1with C, and A corresponding to position 103 of SEQ ID NO:1 with C,

replacing G corresponding to position 101 of SEQ ID NO:1 with C, and V corresponding to position 116 of SEQ ID NO:1 with C,
replacing R corresponding to position 271 of SEQ ID NO:1 with C, and E corresponding to position 413 of SEQ ID NO:1 with C,
replacing R corresponding to position 353 of SEQ ID NO:1 with C, and L corresponding to position 401 of SEQ ID NO:1 with C,
replacing A corresponding to position 147 of SEQ ID NO:1 with C, and Y corresponding to position 268 of SEQ ID NO:1 with C,
replacing G corresponding to position 65 with R,
replacing E corresponding to position 282 of SEQ ID NO: 1 with L,
replacing G corresponding to position 365 of SEQ ID NO: 1 with D,
replacing D corresponding to position 133 of SEQ ID NO: 1 with E,
replacing R corresponding to position 382 of SEQ ID NO: 1 with I, and
replacing S corresponding to position 393 of SEQ ID NO: 1 with I.

Another aspect of the present invention is to provide a DNA construct comprising the polynucleotide comprising a nucleotide sequence encoding the above phytase mutant.
Another aspect of the present invention is to provide an isolated recombinant cell comprising the above polynucleotide.
Another aspect of the present invention is to provide a method of producing the phytase comprising the steps of transforming an isolated host cell with a DNA construct comprising a polynucleotide which comprises a nucleotide sequence encoding said phytase mutant to obtain a recombinant host cell; cultivating the recombinant host cell to produce the phytase mutant; and recovering the phytase.

The present invention introduces a series of mutations to the phytase APPAmut4, which may involve introducing disulfide bonds, reducing the free energy of unfolding, optimizing the key residues in the coevolution process, and significantly improving the thermal stability of the phytase. Among the mutants of the present invention, the optimal mutant APPAmut9 retains about 70% of its activity after being treated for 5 minutes at 100 °C, while the phytase APPAmut4 has already been inactivated. Therefore, the present invention overcomes the shortcomings of the prior art and provides phytase mutants with high thermal stability suitable for wide application in fields such as energy, food, and feed.

## BRIEF DESCRIPTIONS OF THE DRAWINGS

Fig. 1 shows the comparison of the optimal temperatures between the phytase APPAmut4 and its single site and double site mutants;

Fig. 2 shows the comparison of the optimal temperatures between the phytase APPAmut4 and its multi-site mutants.

Fig. 3 shows the comparison of the thermal stability of each single site and double site mutant after being treated at 65 °C;

Fig. 4 shows the comparison of the thermal stability of the phytase APPAmut4 and its multi-site mutants after being treated at 65 °C;

Fig. 5 shows the comparison of $t_{1/2}$ of the phytase APPAmut4 and its mutants.

## EMBODIMENT

Test materials and reagents:

1. Strains and vectors: *Pichia pastoris* GS115 and expressing vector pPICZαA;
2. Enzymes and other biochemical reagents: Endonucleases;
3. Medium:

(1) Low-salt LB (LLB) for *Escherichia coli* :1% peptone, 0.5% yeast extract, 0.5% NaCL, pH natural),
(2) *Pichia pastoris* YPD medium:1% yeast extract, 2% peptone, 2% glucose, 7.0;
(3) BMGY medium: 1% yeast extract, 2% peptone, 1% glycerol, 1.34%YNB, 0.00004%Biotin, pH 7.0;
(4) BMMY medium: 1% yeast extract, 2% peptone, 0.5% methanol,1%, 1.34%YNB, 0.00004%Biotin, pH 7.0.

Suitable biology laboratory methods not particularly mentioned in the examples as below can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989), and other kit laboratory manuals.
Method for determining phytase activity: Diluting the enzyme solution with 0.1 mol/L HAc NaAc buffer containing 0.05% BSA and 0.05% Triton X-100 at pH 5.5, adding 100 μL of the diluted enzyme solution to 900 μL of sodium phytate substrate being prepared with 0.1 mol/L HAc NaAc buffer at pH 5.5, reacting at 37 °C for 10 minutes, adding 1 mL of 10% (W/V) TCA to terminate the reaction, adding 1 mL of colorimetric solution containing 1% (W/V) ammonium molybdate tetrahydrate, 3.2% (V/V) concentrated sulfuric acid, 7.32% (W/V) ferrous sulfate for color development, while the control is added with TCA and mixed well before adding the enzyme solution to denature the enzyme, and measuring the OD value under 700 nm light absorption and calculating the enzyme activity.

## Example 1 Site-directed mutation of the phytase APPAmut4

The site directed mutations were performed with the primers as list in the table 1 using the mutagenesis kit.
Firstly, the amino acid sequence of phytase APPAmut4 derived from *Yersinia intermedia* was performed the mutations of replacing A corresponding to position 57 of SEQ ID NO:1 with C, and A corresponding to position 103 of SEQ ID NO:1 with C to obtain the mutant A57C/A103C, or

replacing G corresponding to position 101 of SEQ ID NO:1 with C, and V corresponding to position 116 of SEQ ID NO:1 with C to to obtain the mutant G101C/V116C, or,
replacing R corresponding to position 271 of SEQ ID NO:1 with C, and E corresponding to position 413 of SEQ ID NO:1 with C to obtain the mutant R271C/E413C, or,
replacing R corresponding to position 353 of SEQ ID NO:1 with C, and L corresponding to position 401 of SEQ ID NO:1 with C to obtain the mutant R353C/L401C, or,
replacing A corresponding to position 147 of SEQ ID NO:1 with C, and Y corresponding to position 268 of SEQ ID NO:1 with C to obtain the mutant R353C/L401C.

Secondly, The amino acid sequence of phytase APPAmut4 derived from *Yersinia intermedia* was performed the mutations of replacing G corresponding to position 65 with R to obtain the mutant G65R, or,

replacing E corresponding to position 282 of SEQ ID NO: 1 with L to obtain the mutant E282L, or,
replacing G corresponding to position 365 of SEQ ID NO: 1 with D to obtain the mutant G365D,
replacing D corresponding to position 133 of SEQ ID NO: 1 with E to obtain the mutant D133E, or
replacing R corresponding to position 382 of SEQ ID NO: 1 with I to obtain the mutant R382I, or,
replacing S corresponding to position 393 of SEQ ID NO: 1 with I to obtain the mutant S393I.

Thirdly, the amino acid sequence of phytase APPAmut4 derived from *Yersinia intermedia* was performed the mutations of replacing A corresponding to position 57, A corresponding to position 103, G corresponding to position 101, V corresponding to position 116, R corresponding to position 271, E corresponding to position 413, R corresponding to position 353, L corresponding to position 401, A corresponding to position 147, Y corresponding to position 268 of SEQ ID NO: 1 with C respectively, to obtain the mutant APPAmut5.

Fourthly, the amino acid sequence of the APPAmut5 was performed the additional mutations of replacing G corresponding to position 65 with R, E corresponding to position 282 with L, and G corresponding to position 365 with D, using the primers for the site directed mutagenesis, to obtain the mutant APPAmut8 .

Fifthly, the amino acid sequence of the APPAmut8 was performed the additional mutations of replacintg D corresponding to position 133 with E, R corresponding to position 382 with I, and S corresponding to position 393 with I using the primers for the site directed mutagenesis to obtain the mutant APPAmut9.

Table 2 primers for the site directed mutagenesis

| Primer | Sequence of the primers (5'-3') |
|---|---|
| A147C-F | ACCCACAAATGTGTTGAAGAGAG |
| Y268C-R | TCTAGCAATACAAGGAGTCTTTG |
| R271C-F | TACATTGCTTGTCACAAAGGAACTC |
| E413C-R | ACAAGCTGGACATATGGCCTGAGCAACC |
| G101C-R | TCCTGGAGCGATACCATCCAAGAATGCTTGACAGGTCAAACGAGTTC |
| V116C-F | TTCTTGGATGGTATCGCTCCAGGATGTGGCTTGAAATGTCACTACCAGG |
| A57C-F | CACCAAGAGGTTGTCAGTTGGTTAC |
| A103C-R | CATCCAAGAAACATTGTCCGGTCAAAC |
| R353C-F | CAACCACCAGTGTTACGTTGCTGTAAAG |
| L401C-R | GAAAGTATCACACTGGCACAAC |
| R353C-R | AGCAACGTAACACTGGTGGTTGTCAGGGTTTTGC |
| L401C-F | GCCAGTGTGATACTTTCCAGAAGAAGG |
| G365D-F | CAGACTATGGATCAATTGCGTAACGCAGAG |
| G365D-R | CGCAATTGATCCATAGTCTGATAGAACAAC |
| A89V-F | GACATCTACGTTCAAGCTGACGTTGATCAAAG |
| A89V-R | GTCAGCTTGAACGTAGATGTCAGCATCAGTAG |
| G65R-F | ATGGGTAGATTCTACGGTGACTATTTCAG |
| G65R-R | ACCGTAGAATCTACCCATCAAAGTAACCAAC |
| E282L-F | CAGCAAATCTTGACTGCTTTGGTCCTCCAAAG |
| E282L-R | CAAAGCAGTCAAGATTTGCTGAAGCAAGGGAG |

(continued)

| Primer | Sequence of the primers (5'-3') |
| --- | --- |
| D133E-F | CACCCTGTTGAAGCAGGTGTTTGTAAGCTTG |
| D133E-R | AAACACCTGCTTCAACAGGGTGGAACAGTGG |
| R382I-F | CCAGCCGGTATTGTTCCTGTCGCAATTGACG |
| R382I-R | GACAGGAACAATACCGGCTGGGTTGTTCTTC |
| S393I-F | TGTGAGAACATTGGAGATGACAAGTTGTGCC |
| S393I-R | GTCATCTCCAATGTTCTCACAACCGTCAATTG |

•

## Example 2 Constructing the strain expressing the phytase APPAmut4 and its mutants

PCR amplification was performed using primers containing the corresponding mutation sites and plasmid pPICZαA-*appamut4* as the template, and then the PCR amplification product was analyzed by 1% agarose gel electrophoresis, wherein If the size of the band was consistent with the theoretical value, the PCR reaction was successful in obtaining the target product. In order to eliminate the interference of template plasmids on subsequent experiments, 1 μL of restriction enzyme Dpn I was added to the PCR system based on the methylation difference between template plasmids and PCR products, followed by being digested at 37 °C for 1 to 2 hours. Then, 10 μL of the product was transformed into *E. coli* DMT competent cells, followed by extracting the recombinant plasmid when the result of sequencing is correct, linearizing it using the restriction enzyme *Pme* I, purifying and recovering the product, and transforming it into the competent cells of *Pichia pastoris* GS115 to obtain the recombinant expression strain of *Pichia pastoris.*

## Example 3 Preparing the phytase APPAmut4 and its mutant

The obtained recombinant expression strain was inoculated into YPD medium for seed culture at 200 rpm and 30°C for 48 hours, followed by being transferred to BMGY medium in 1% inoculation amount and cultured at 200 rpm and 30 °C for 48 hours. Then, the supernatant was discard after being centrifuged at 4500 rpm for 5 minutes, the bacterial cells were collected, and BMMY medium containing 0.5% of methanol was added for inducing the expression with a addition of 0.5% of methanol every 12 hours for a total of 48 hours of induction.

The induced bacterial solution was centrifuged at 12000 rpm for 10 minutes, to collect the supernatant for concentration, followed by being dialyzed with 20 mM of Tris HCl at pH 8.0. Then, the dialyzed enzyme solution was subjected to anion exchange chromatography, with the solution A containing 20 mM of Tris HCL at pH 8.0 and the solution B obtained by adding 1 M of NaCL to the solution A, to putify the protein, and the eluent was collected for SDS-PAGE analysis.

## Example 4 Measuring the enzymatic properties of mutants

(1) Determining the optimal temperature

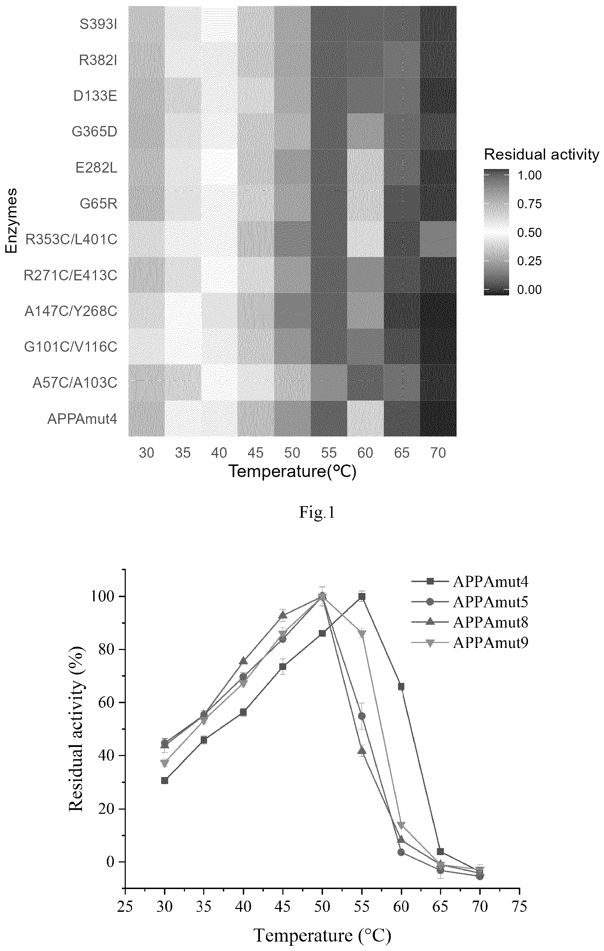

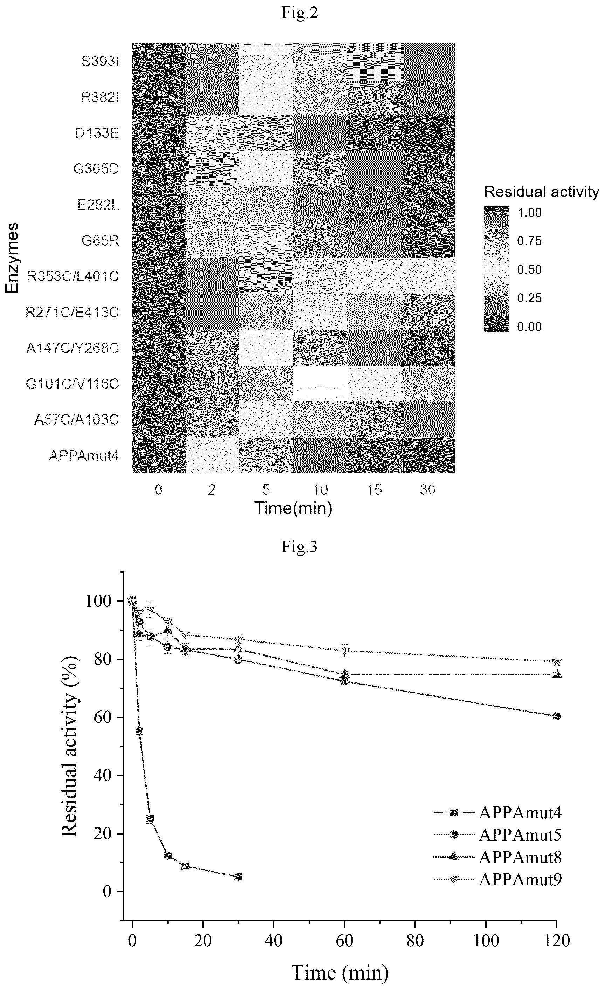

The enzyme activity of wild-type and mutant was measured at the different temperatures of 30°C , 35°C, 40 °C, 45 °C, 50°C, 55 °CC, 60 °C, 65 °C, and 70 °C with 0.1 mol/L of HAc NaAc buffer at pH to determine the optimal temperature, wherein the activity corresponding to the optimal temperature was defined as 100%, and the remaining enzyme activity at the other temperatures was calculated. As shown in Figure 1, the optimal temperature for APPAmut4 is 55 °C, while the optimal temperature for mutant A57C/A103C increases to 60 °C, the optimal temperature for L253C/P331C decreases to 50°C, and the other mutants remain unchanged. However, the enzyme activity of the mutants D133E, R382I, and S393I at 60 °C is almost the same as that at 55 °C, indicating that all three mutants have good catalytic function between 55 and 60 °C. As shown in Figure 2, the optimal temperature for APPAmuT5, APPAmuT8, and APPAmuT9 is 50 °C.

(2)Measuring the thermal stability

The purified protein was diluted with 0.1 mol/L of HAc NaAc buffer in pH 5.5 containing 0.05% BSA and 0.05% Triton X-100 to an appropriate multiple, followed by taking 100 μL for being incubated at 65°C for 0, 2, 5, 10, 15, and 30 min respectively, and then measuring the corresponding enzyme activity, wherein the activity at 0 min is considered as 100%, and the remaining enzyme activities at different incubation time were calculated respectively.

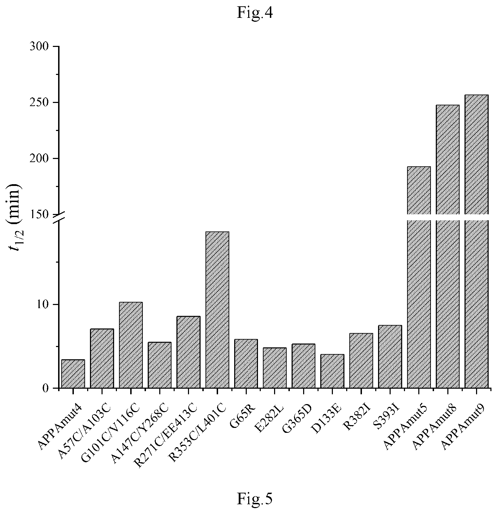

As shown in Figure 3, after being treated at 65°C for 10 minutes, the remaining enzyme activity of APPAmut4 was 12.3%, while the remaining enzyme activity of each double point and single point mutant ranged from 13.6% to 64.4%; As shown in Figure 4, the residual enzyme activities of the mutants APPAmut5, APPAmut8, and APPAmut9 after being treated at 65°C for 10 minutes were 84.3%, 89.9%, and 93.2%, respectively.

Half life $t_{1/2}$ refers to the time that takes for the initial activity to decrease by 50% at a given temperature, and was calculated by the following formula,

$$t_{1/2} = \frac{\ln 2}{k_d},$$

wherein $k_d$ is the deactivation rate constant, which can be obtained through the following linear regression:

$$\ln \frac{A_t}{A_0} = -k_d t,$$

wherein At refers to the residual activity, $A_0$ is the initial activity, and t is the processing time at the studied temperatures.

As shown in Figure 5, the value of $t_{1/2}$ of the phytaset APPAmut4 at 65 °C is 3.4 min, while the values of $t_{1/2}$ of each double point and single point mutant are ranging from 4.0 to 18.6 min, increased by from 0.6 to 15.2 min; and the values of $t_{1/2}$ of the combined mutants APPAmut5, APPAmut8, and APPAmut9 at 65°C were 192.5, 247.6, and 256.7 min, respectively, which were 56, 72, and 74 times higher than that of the phytase APPAmut4, respectively. In practical applications, the life of enzyme is significantly correlated with its kinetic stability, i.e. the time or temperature required for proteins to maintain partial activity during irreversible denaturation. The half-life $t_{1/2}$ is a conventional parameter for characterizing the kinetic stability of enzymes, and the larger its value, the higher the kinetic stability of the enzyme. The test measurements showed that the value of $t_{1/2}$ of the mutant increased to the varying degrees as compared to the phytase APPAmut4 through experimental measurements, indicating that its thermal stability was improved to varying degrees. The value of $t_{1/2}$ of the mutant APPAmut9 is as high as 256.7 minutes, indicating a significant improvement in thermal stability.

(3)Determination of dynamic parameters

The different concentrations of sodium phytate ranging from 0.05 to 1.00 mM were prepared as substrates and the activity of phytase was measured at 37°C and pH 5.5, wherein the software GraphPad Prism was used for data processing, and the values of $K_m$ and $k_{cat}$ were calculated. As shown in Table 2, the value of $K_m$ of the phytase APPAmut4 is 0.14 mM, while the values of $K_m$ of the mutants A57C/A103C, G101C/V16C, A147C/Y268C, R271C/E413C, R353C/L401C, and G65R have increased in the varying degrees ranging from 0.18 to 0.22, indicating a decrease in substrate affinity. The catalytic efficiency $k_{cat}/K_m$ of the phytase APPAmut4 was 12322/mM/s, while the $k_{cat}/K_m$ of the mutants A147C/Y268C and G65R decreased to 10823 and 9009/mM/s, respectively, indicating that the mutation affected its catalytic activity, and the catalytic efficiency of the remaining single point and double point mutants remains unchanged or slightly improves. The $k_{cat}/K_m$ for the combination mutants APPAmut5, APPAmut8, and APPAmut9 are 11632, 13018, and 11537/mM/s, which remained relatively unchanged as compared to the phytase APPAmut4, respectively, indicating that a significant the improvement in thermal stability didn't reduce their catalytic efficiency.

Table 2 Dynamics parameters of the phytase APPAmut4 and its mutants

| Enzymes | $K_m$ (mM) | $V_{max}$ (μmol/min/mg) | $k_{cat}$ (/s) | $k_{cat}/K_m$ (/mM/s) |
|---|---|---|---|---|
| APPAmut4 | 0.14±0.03 | 2272±95 | 1725±72 | 12322±1577 |
| A57C/A103C | 0.18±0.03 | 2845±171 | 2160±130 | 12068±1368 |
| G101C/V116C | 0.20±0.03 | 2932±157 | 2226±119 | 11131±1132 |
| A147C/Y268C | 0.22±0.03 | 3136±163 | 2381±124 | 10823±1019 |
| R271C/E413C | 0.18±0.03 | 3177±203 | 2412±154 | 13402±1545 |
| R353C/L401C | 0.20±0.02 | 3400±163 | 2581±124 | 12908±955 |
| G65R | 0.22±0.04 | 2634±101 | 2000±77 | 9009±992 |
| E282L | 0.15±0.02 | 2300±88 | 1746±67 | 11269±967 |
| G365D | 0.14±0.02 | 2411±76 | 1831±58 | 13158±1148 |
| D133E | 0.13±0.03 | 2477±76 | 1881±58 | 14989±1960 |

(continued)

| Enzymes | $K_m$ (mM) | $V_{max}$ (μmol/min/mg) | $k_{cat}$ (/s) | $k_{cat}/K_m$ (/mM/s) |
|---------|-----------|------------------------|----------------|------------------------|
| R382I | 0.16±0.02 | 2564±106 | 1947±80 | 11958±993 |
| S393I | 0.14±0.03 | 2772±126 | 2105±96 | 14716±1912 |
| APPAmut5 | 0.15±0.02 | 2298±108 | 1744±82 | 11632±1048 |
| APPAmut8 | 0.15±0.02 | 2630±127 | 1997±96 | 13018±1181 |
| APPAmut9 | 0.15±0.02 | 2279±134 | 1730±102 | 11537±1109 |

The above embodiments are only used to illustrate the technical solution of the present application and do not limit the scope of protection of the present application.

**Claims**

1. A phytase mutant having phytase activity and comprising the amino acid sequence of SEQ ID NO:1, except A corresponding to position 57 and A corresponding to position 103 of SEQ ID NO: 1 are replaced with C respectively, or,

    G corresponding to position 101 and V corresponding to position 116 of SEQ ID NO: 1 are replaced with C respectively, or
    R corresponding to position 271 and E corresponding to position 413 of SEQ ID NO: 1 are replaced with C respectively, or
    R corresponding to position 353 and L corresponding to position 401 of SEQ ID NO: 1 are replaced with C respectively, or
    A corresponding to position 147 and Y corresponding to position 268 of SEQ ID NO: 1 are replaced with C respectively, and wherein the phytase mutant has the improved thermal stability as compared to a phytase consisting of the amino acid sequence of SEQ ID NO: 1.

2. The phytase mutant according to claim 1, wherein said phytase mutant comprises the amino acid sequence of SEQ ID NO:1, further except that G corresponding to position 65 is replaced with R, or,

    E corresponding to position 282 of SEQ ID NO: 1 is replaced with L or,
    G corresponding to position 365 of SEQ ID NO: 1 is replaced with D, or
    D corresponding to position 133 of SEQ ID NO: 1 is replaced with E, or
    R corresponding to position 382 of SEQ ID NO: 1 is replaced with I, or,
    S corresponding to position 393 of SEQ ID NO: 1 is replacedwith I

3. The phytase mutant according to claim 1 or 2, wherein said phytase mutant comprises the amino acid sequence of SEQ ID NO:2, or SEQ ID NO:3, or SEQ ID NO:4.

4. A polynucleotide comprising a nucleotide sequence encoding the phytase mutant of any one of claims 1 to 3.

5. A DNA construct comprising the polynucleotide of claim 4.

6. An isolated recombinant cell comprising the polynucleotide of claim 4.

7. A method of improving the thermal stability of a phytase comprising an amino acid of SEQ ID NO:1, wherein said method comprising the step of

    replacing A corresponding to position 57 of SEQ ID NO:1with C, and A corresponding to position 103 of SEQ ID NO:1 with C, or,
    replacing G corresponding to position 101 of SEQ ID NO:1 with C, and V corresponding to position 116 of SEQ ID NO:1 with C, or,
    replacing R corresponding to position 271 of SEQ ID NO:1 with C, and E corresponding to position 413 of SEQ ID NO:1 with C, or,
    replacing R corresponding to position 353 of SEQ ID NO:1 with C, and L corresponding to position 401 of SEQ ID NO:1 with C, or,

replacing A corresponding to position 147 of SEQ ID NO:1 with C, and Y corresponding to position 268 of SEQ ID NO:1 with C.

8. The method according to claim 7, wherein said method further comprises the step of replacing G corresponding to position 65 with R, or,

replacing E corresponding to position 282 of SEQ ID NO: 1 with L, or,
replacing G corresponding to position 365 of SEQ ID NO: 1 with D, or,
replacing D corresponding to position 133 of SEQ ID NO: 1 with E, or,
replacing R corresponding to position 382 of SEQ ID NO: 1 with I, or,
replacing S corresponding to position 393 of SEQ ID NO: 1 with I.

9. The method according to claim 7, wherein said method comprises the step of replacing A corresponding to position 57, A corresponding to position 103, G corresponding to position 101, V corresponding to position 116, R corresponding to position 271, E corresponding to position 413, R corresponding to position 353, L corresponding to position 401, A corresponding to position 147, and Y corresponding to position 268 of SEQ ID NO:1 with C. respectively.

10. The method according to claim 9, wherein said method further comprises the steps of replacing G corresponding to position 65 with R, replacing E corresponding to position 282 of SEQ ID NO: 1 with L, and replacing G corresponding to position 365 of SEQ ID NO: 1 with D.

11. The method according to claim 10, wherein said method further comprises the steps of replacing D corresponding to position 133 of SEQ ID NO: 1 with E, replacing R corresponding to position 382 of SEQ ID NO: 1 with I, and replacing S corresponding to position 393 of SEQ ID NO: 1 with I.

12. A use of the phytase mutant of any one of claims 1 to 3 tohydrolyzing the phytic acid.

Fig.1

Fig.2

Fig.3

Fig.4

Fig.5

<div align="center">

**INTERNATIONAL SEARCH REPORT**

</div>

| | International application No. |
|---|---|
| | **PCT/CN2023/073753** |

**A.    CLASSIFICATION OF SUBJECT MATTER**

C12N9/16(2006.01)i;C12N15/55(2006.01)i;C12N15/81(2006.01)i;C12N1/19(2006.01)i;C12R1/84(2006.01)i

According to International Patent Classification (IPC) or to both national classification and IPC

**B.    FIELDS SEARCHED**

Minimum documentation searched (classification system followed by classification symbols)

C12N

Documentation searched other than minimum documentation to the extent that such documents are included in the fields searched

Electronic data base consulted during the international search (name of data base and, where practicable, search terms used)

VEN, CNABS, PubMed, ISI Web of Science, VCN, CNKI, 万方, WANFANG, 百度学术, BAIDU SCHOLAR, 读秀, DUXIU, STN, GenBank, 中国专利生物序列检索系统, China Patents Biological Sequence Search System: 植酸酶, phytase, 突变体, R271, E413, L40, G65, E282, G365, muatein, mutatant, SEQ ID NOs: 1-4.

**C.    DOCUMENTS CONSIDERED TO BE RELEVANT**

| Category* | Citation of document, with indication, where appropriate, of the relevant passages | Relevant to claim No. |
|---|---|---|
| PX | CN 114807087 A (INSTITUTE OF ANIMAL SCIENCES OF CHINESE ACADEMY OF AGRICULTURAL SCIENCES) 29 July 2022 (2022-07-29)<br>entire document | 1-12 |
| X | CN 102597227 A (BIOMETHODES) 18 July 2012 (2012-07-18)<br>claims 1-15, and description, paragraphs 118-145, and sequence listing, sequences 1-2 | 1-12 |
| A | CN 106434595 A (FEED RESEARCH INSTITUTE, CHINESE ACADEMY OF AGRICULTURAL SCIENCES) 22 February 2017 (2017-02-22)<br>entire document | 1-12 |
| A | CN 101426907 A (FEED RESEARCH INSTITUTE, CHINESE ACADEMY OF AGRICULTURAL SCIENCES) 06 May 2009 (2009-05-06)<br>entire document | 1-12 |
| A | WO 2017215218 A1 (KUMING QACTIVE BIO-SCIENCES CO., LTD.) 21 December 2017 (2017-12-21)<br>entire document | 1-12 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **03 April 2023** | **21 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

International application No.

**PCT/CN2023/073753**

**C.    DOCUMENTS CONSIDERED TO BE RELEVANT**

| Category* | Citation of document, with indication, where appropriate, of the relevant passages | Relevant to claim No. |
|---|---|---|
| A | WO 2007128160 A1 (FEED RESEARCH INSTITUTE, CHINESE ACADEMY OF AGRICULTURAL SCIENCES, et al.) 15 November 2007 (2007-11-15)<br>entire document | 1-12 |
| A | 陈惠等 (CHEN, Hui et al.). "F43Y及I354M,L358F定点突变对植酸酶热稳定性及酶活性的改善 (Site-directed Mutagenesis Improves the Thermostability and Specific-activity of Phytase Mutated by F43Y and I354M, L358F)"<br>*中国生物化学与分子生物学报 (Chinese Journal of Biochemistry and Molecular Biology)*, Vol. 21, No. 4, 15 August 2005 (2005-08-15),<br>516-520 | 1-12 |
| A | Huang, H. et al. "phytase [Yersinia intermedia]"<br>*GenBank: ABI95370.1*, 14 July 2016 (2016-07-14),<br>origin | 1-12 |

**INTERNATIONAL SEARCH REPORT**

International application No.

**PCT/CN2023/073753**

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

a. [✓]  forming part of the international application as filed.

b. [ ]  furnished subsequent to the international filing date for the purposes of international search (Rule 13*ter*.1(a)),

[ ]  accompanied by a statement to the effect that the sequence listing does not go beyond the disclosure in the international application as filed.

2. [ ]  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

INTERNATIONAL SEARCH REPORT
Information on patent family members

International application No.

**PCT/CN2023/073753**

| Patent document cited in search report | | | Publication date (day/month/year) | Patent family member(s) | | | Publication date (day/month/year) |
|---|---|---|---|---|---|---|---|
| CN | 114807087 | A | 29 July 2022 | None | | | |
| CN | 102597227 | A | 18 July 2012 | FR | 2947563 | A1 | 07 January 2011 |
| | | | | FR | 2947563 | B1 | 21 August 2015 |
| | | | | US | 2013122567 | A1 | 16 May 2013 |
| | | | | US | 9109210 | B2 | 18 August 2015 |
| | | | | EP | 2449100 | A2 | 09 May 2012 |
| | | | | WO | 2011000933 | A2 | 06 January 2011 |
| | | | | WO | 2011000933 | A3 | 17 March 2011 |
| | | | | CA | 2766704 | A1 | 06 January 2011 |
| | | | | CA | 2766704 | C | 12 June 2018 |
| CN | 106434595 | A | 22 February 2017 | None | | | |
| CN | 101426907 | A | 06 May 2009 | US | 2010092613 | A1 | 15 April 2010 |
| | | | | US | 8455620 | B2 | 04 June 2013 |
| | | | | EP | 2021466 | A1 | 11 February 2009 |
| | | | | EP | 2021466 | A4 | 09 June 2010 |
| | | | | EP | 2021466 | B1 | 29 August 2012 |
| | | | | JP | 2009535043 | A | 01 October 2009 |
| | | | | JP | 5340138 | B2 | 13 November 2013 |
| | | | | DK | 2021466 | T3 | 26 November 2012 |
| | | | | WO | 2007128160 | A1 | 15 November 2007 |
| WO | 2017215218 | A1 | 21 December 2017 | None | | | |
| WO | 2007128160 | A1 | 15 November 2007 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Molecular Cloning: A Laboratory Manual.. **SAMBROOK et al.** Cold Spring Harbor Laboratory,. Cold Spring Harbor Laboratory Press, 1989